# EUROPEAN PATENT SPECIFICATION

(11) **EP 1 463 488 B1**
(45) Date of publication and mention of the grant of the patent: **19.07.2006**
(21) Application number: 02788203.4
(22) Date of filing: 19.12.2002
(51) Int. Cl.: A61K 9/20

(54) **PHARMACEUTICAL COMPOSITION COMPRISING SKIMMED MILK POWDER**
PHARMAZEUTISCHE ZUSAMMENSETZUNG ENTHALTEND MAGERMILCHPULVER
COMPOSITION PHARMACEUTIQUE COMPRENANT DE LA POUDRE DE LAIT ECREME

(30) Priority: 20.12.2001 US 343127 P
(43) Date of publication of application: 06.10.2004
(73) Proprietor: Alpex Pharma SA, 6805 Mezzovico-vica (CH)
(72) Inventor: STROPPOLO, Federico, CH-6974 Aldesago (CH); CICCARELLO, Franco, CH-6809 Mezzovico (CH); MILANI, Rita, CH-6952 Canobbio (CH); BELLORINI, Lorenzo, I-21028 Travedona(Monate) (IT)
(74) Representative: van Wijk, Alexander Pieter
(86) International application number: PCT/GB2002/005825
(87) International publication number: WO 2003/053415

(56) References cited:
- WO-A-98/34607
- GB-A- 932 378
- US-A- 3 590 117
- US-A- 5 785 984

## Description

### FIELD OF THE INVENTION

The present invention relates to pleasant-tasting compositions made from an unpleasant-tasting therapeutic agent and a taste-masking agent, oral dosage forms of such compositions, methods of making the compositions and methods of treating therewith.

Many dosage forms of therapeutic agents are designed to be administered orally. This route of administration is convenient, economical and effective in quickly and easily placing the desired dosage of a therapeutic agent in contact with the relatively large surface membrane of the stomach which has a rich supply of capillaries for passage into the bloodstream. A common disadvantage associated with many such oral dosage forms is the presence of an objectionable or unpleasant taste. The presence of an objectionable or unpleasant taste can affect patient compliance adversely, especially for therapeutic agents in which an extended or frequent course and/or large doses are indicated. Thus, it has been recognized that improving the taste of unpleasant-tasting therapeutic agents can affect patient compliance positively.

### REPORTED DEVELOPMENTS

Compositions have been developed for the purposes of improving the taste of unpleasant-tasting therapeutic agents. For example, British Patent No. 1,257,594 to the Lepetit Group (hereinafter "the '594 patent") discloses a composition comprising aluminum sodium silicate and powdered milk. The composition is prepared by suspending aluminum sodium silicate and powdered milk in water and then drying the suspension by evaporation *in vacuo* either by heating the mixture under reduced pressure or by lyophilization. The composition formed in accordance with this process is disclosed as distinctly different from a composition of aluminum sodium silicate and powdered milk formed by dry blending insofar as the dry blended composition is not considered to be effective in masking the unpleasant taste of aluminum sodium silicate.

U.S. Patent No. 5,785,984 to Kurihara et. al. (hereinafter "the '984 patent") discloses a taste-modifying, bitterness-masking and bitterness-decreasing agent comprising a protein-lipid complex which can be added to a food, pharmaceutical or cosmetic that has a bitter-tasting component. The compositions of the '984 patent are produced by a relatively complicated process in which the protein-lipid complex is itself created in a multi-step process and then combined with a food, pharmaceutical or cosmetic that has a bitter-tasting component.

British Patent No. 932,378 discloses antacid preparations made by spray-drying comprising drug particles coated with a film of the solid content of skimmed milk or whole milk for taste masking.

### SUMMARY OF THE INVENTION

In accordance with the present invention, there is provided a pharmaceutical composition comprising therapeutic agent - generally speaking an unpleasant-tasting one - and skim milk powder. The agent may be any substance or mixture of substances intended for oral administration that has curative, beneficial and/or nutritional value in humans. Typically it has a substantially stable unpleasant taste. The skim milk powder suitable for use may be any commercially available milk powder, or synthetic equivalent thereof, usually having a fat content of less than about 1.25 wt.% and a moisture content of less than about 4 wt.%,. The composition of the present invention, preferably in oral dosage form, may further comprise a conventional pharmaceutical excipient. The excipient can serve a number of functions, for example, further enhancing the taste of the composition and/or contributing to the physical properties of oral dosage forms made therefrom.

A further aspect of the present invention is a method of making a pharmaceutical composition comprising the step of dry blending such therapeutic agent and skim milk powder in an amount sufficient to mask the (unpleasant) taste of the therapeutic agent and tableting the resulting mixture. The therapeutic agent and the skim milk powder are combined in a ratio sufficient to provide effective taste-masking of the therapeutic agent, preferably in a ratio of skim milk powder to therapeutic agent of from at least 1:1, typically from about 1:1 to about 1,000,000:1.

There are important advantages that stem from the formulation and use of the present invention. The use of skim milk powder is effective in masking the taste of many unpleasant-tasting therapeutic agents. It is also an inexpensive, pharmaceutically acceptable and readily available material that can contribute positively to the physical properties required of many oral dosage forms. Further, compositions of the present invention are able to be formed by conventional and inexpensive production techniques. Such compositions are suitable for use in the formulation of a wide variety of oral dosage forms.

### DETAILED DESCRIPTION OF THE INVENTION

The composition of the present invention comprises a therapeutic agent and skim milk powder.

The term "therapeutic agent" as relevant for the purposes of the present invention includes any substance or mixture of substances intended for oral administration that has curative, beneficial and/or nutritional value in humans and which has a substantially unpleasant taste, either alone or in combination with other substances. The term "unpleasant taste" is defined for present purposes as a taste which gives rise to a statistically significant rating of possessing an objectionable taste when the unmasked form of the therapeutic agent having such taste is provided in a random sampling to a group of humans having an ordinary perception of taste. Examples of such therapeutic agents are set forth in table 1 below.

**TABLE 1**

| Category | Agent | Typical Dosage Range |
|---|---|---|
| ACE Inhibitor | Enalapril | 2.5 mg - 20 mg |
| | Captopril | 12.5 mg - 50 mg |
| | Ramipril | 2.5 mg - 5 mg |
| Analgesic | Acetaminophen | 100 mg - 500 mg |
| Antidepressant | Imipramine | 10 mg - 50 mg |
| | Citalopram | 20 mg |
| | Fluexitine | 10 mg - 20 mg |
| Antihistaminic | Astemizole | 5 mg - 10 mg |
| | Cetirizine | 5 mg -10 mg |
| | Clemastine | 1.34 mg - 2.68 mg |
| | Ebastine | 10 mg |
| | Fexofenadine | 30 mg -180 mg |
| | Loratadine | 10 mg |
| Antiulcerative | Famotidine | 20 mg - 40 mg |
| | Cimetidine | 20 mg - 800 mg |
| | Omeprazole | 10 mg - 40 mg |
| Anti-arrhythmic | Acebutolol | 200 mg - 400 mg |
| | Amiodarone | 100 mg - 200 mg |
| | Atenolol | 25 mg - 100 mg |
| | Carteolol | 5 mg - 20 mg |
| | Verapamil | 120 mg - 240 mg |
| Beta - adrenergic agonist | Albuterol | 2 mg - 4 mg |
| | Dopexamine | 50 mg |
| | Formoterol | 6 mg - 12 mg |
| Calcium antagonist | Diltiazem | 60 mg - 300 mg |
| | Lacidipine | 2 mg - 4 mg |
| | Nicardipine | 20 mg |
| | Nifedipine | 5 mg - 20 mg |
| | Verapamil | 20 mg - 400 mg |
| Corticosteroid | Budesonide | 200 mg - 400 mg |
| | Dexomethasone | 0.5 mg - 10 mg |
| | Prednisolone | 5 mg - 15 mg |
| COX-2 Inhibitor | Rofecoxib | 12.5 mg - 25 mg |
| | Celecoxib | 100 mg - 200 mg |
| Decongestant | Phenyleprine | 1.25 mg - 20 mg |
| Energy promoter | Carnitine | 50 mg - 500 mg |
| | Ubidecarenone | 2 mg - 100 mg |
| Expectorant | Guaifenesin | 50 mg -1200 mg |
| Lipid regulating drug | Lovastatine | 10 mg - 20 mg |
| Mineral salt | Boron | 20 mg - 200 mg |
| | Calcium | 50 mg - 2000 mg |
| | Chloride | 20 mg -100 mg |
| | Chromium | 0.02 mg - 0.2 mg |
| | Copper | 0.6 mg - 5 mg |
| | Iodine | 20 mg - 200 mg |
| | Iron | 2 mg - 20 mg |
| | Magnesium | 10 mg -1000 mg |
| | Manganese | 0.6 mg - 10 mg |
| | Molybdenum | 20 mg - 200 mg |
| | Nickel | 0.5 mg - 20 mg |
| | Potassium | 20 mg -1000 mg |
| | Phosphorus | 50 mg -1500 mg |
| | Selenium | 5 mg -100 mg |
| | Silicon | 2 mg - 20 mg |
| | Tin | 5 mg -100 mg |
| | Vanadium | 5 mg - 100 mg |
| | Zinc | 0.6 mg - 100 mg |
| Mucolytic | Ambroxol | 30 mg - 60 mg |
| | Carbocisteine | 200 mg - 275 mg |
| | N-Acetylcysteine | 50 mg - 1000 mg |
| | Sobrerol | 200 mg - 300 mg |
| Natural product | Acerola | 5 mg - 5000 mg |
| | Ginseng | 0.5 mg - 5.000 mg |
| | Guaranà | 5 mg - 500 mg |
| | Lycopene | 5 mg - 5000 mg |
| Narcotic | Fentanyl | 0.05 mg |
| | Morphine | 7.6 mg - 75 mg |
| | Methadone | 5 mg |
| Non-Steroidal | Aceclofenac | 100 mg |
| Anti-Inflammatory Drug | Ibuprofen | 50 mg - 1200 mg |
| | Ketoprofen | 25 mg - 200 mg |
| | Ketorolac | 10 mg |
| | Naproxen | 250 mg - 500 mg |
| | Nimesulide | 50 mg - 100 mg |
| Tranquilizer non narcotic | Buspirone | 5 mg -10 mg |
| | Alprazolam | 0.5 mg - 3 mg |
| | Camazepam | 10 mg |
| | Clorazepam | 11.25 mg - 50 mg |
| | Lorazepam | 1 mg - 2.5 mg |
| | Nitrazolam | 5 mg |
| Vitamin | Vitamin A | 8 mg - 1300 mg |
| | Vitamin B₁ | 0.1 mg - 4 mg |
| | Vitamin B₂ | 0.1 mg - 4 mg |
| | Vitamin B₆ | 0.05 mg - 4 mg |
| | Vitamin B₁₂ | 0.2 mg - 4 mg |
| | Vitamin C | 5 mg - 2000 mg |
| | Vitamin D₃ | 0.5 mg - 20 mg |
| | Vitamin E | 0.5 mg - 20 mg |
| | Vitamin K₁ | 0.5 mg - 200 mg |
| | Vitamin PP | 1 mg - 20 mg |
| | Pantothenic Acid | 1 mg - 10 mg |
| | Biotin | 0.5 mg - 100 mg |
| | Folic Acid | 30 mg -1000 mg |

The invention includes within its scope the use of pharmaceutically acceptable salts of any of the foregoing and mixtures of two or more of any of the foregoing.

Preferred therapeutic agents for use in the composition of the present invention are minerals, vitamins, natural products, antihistaminics, antiuceratives, corticosteroids, antidepressants, mucolytics, expectorants, decongestants, antitussives and analgesics. Particularly preferred therapeutic agents are: calcium; vitamin D₃; acerola; vitamins B₁, B₆, and PP; acerola; ginseng; guaranà; clemastine fumarate; cetirizine; famotidine; prednisolone salts, imipramine, N-acetylcysteine, guaifenesin, phenylephrine, dextromethorpan, acetaminophen, and fexofenadine.

The therapeutic agent may include two or more substances having the same or different beneficial properties may be used. For example, the therapeutic agent may comprise a combination of vitamins, minerals and other dietary supplements in relative proportions suitable for the formation of an oral dosage form that provides substantially all of a person's daily nutritional needs.

The therapeutic agent is included in the composition in an amount sufficient to impart to oral dosage forms made therefrom the desired curative, beneficial and/or nutritional effect. Such amount will vary in accordance with a number of factors including, for example, the particular species of therapeutic agent used, the presence of other ingredients, the specific type of oral dosage formulation employed, and the particular application in which the composition is intended to be used. It is believed that in most applications, the amount of therapeutic agent included in the composition will be from about 0.01 wt.% to about 50.00 wt.%. In preferred form, the amount of therapeutic agent included in the composition will be from about 0.05 wt.% to about 30.00 wt.%, and even more preferably from about 0.09 wt.% to about 8.33 wt.%.

The use of the present invention is particularly effective with a therapeutic agent where unpleasant taste is substantially stable over time, that is, the degree of unpleasantness does not intensify due to chemical changes in the therapeutic agent, for example, through hydrolysis, oxidization, or degradation, of one or more constituents comprising the agent. An example of a therapeutic agent with an unpleasant taste that is not substantially stable over time is acetylsalicylic acid (ASA). In the presence of moisture, ASA is known to hydrolyze into acetic and salicylic acids. The presence of acetic acid resulting from hydrolysis can increase substantially the unpleasant taste of ASA. As a consequence, an amount of skim milk powder which is effective in masking the taste of unhydrolyzed ASA may be rendered less effective or even ineffective in masking the taste of ASA which has hydrolyzed.

As set forth above, the term "skim milk powder" is defined for present purposes as the solids component of skim milk in powder form with no more than about 4 % moisture content and no more than about 1.25% fat content. Skim milk powder is made commonly by spray drying liquid skim milk and comprises typically from about 34% to about 37% protein; from about 49% to about 52% carbohydrate; from about 8% to about 9% ash; minerals, for example, calcium, sodium, potassium, phosphorus, iron, magnesium and zinc; and amino acids, for example, isoleucine, leucine, lysine, methionine and phenylalanine. It is further considered within the scope of the present invention to use in place of or in combination with the skim milk powder a synthetic mixture of some or all of the components found in skim milk powder (e.g. those identified above preferably including milk protein(s)).

The skim milk powder is included in the composition in an amount at least sufficient to mask the taste of the unpleasant-tasting therapeutic agent. An amount of skim milk powder in excess of the amount required to mask the taste of the unpleasant-tasting therapeutic agent may be desired in applications in which other desired properties are sought. The amount of skim milk powder will vary in accordance with a number of factors including, for example, the particular species of therapeutic agent used, the presence of other ingredients, the specific type of the oral dosage formulation, and the particular application in which the composition is intended to be used. It is believed that for most applications, the ratio of skim milk powder to therapeutic agent will be from about 1:1 to about 1,000,000:1. In preferred form, the ratio will be from about 1:1 to about 1,000:1, and even more preferably from about 1:1 to about 100:1. (These are generally weight ratios).

While not intending to be bound by any particular theory, it is believed that the proteins in the skim milk powder act to mask the taste of the therapeutic agent by blocking the taste receptors located on the tongue. This blocking action may occur either by preferential binding of the proteins to receptor sites, by formation of a film over the receptor site which acts as a physical barrier to the therapeutic agent, or by some other mechanism. The carbohydrates and sugars present in skim milk powder enhance the palatability of the compositions and contribute to the physical characteristics of the composition.

The composition of the present invention may include also conventional excipients of the type used in pharmaceutical compositions. Examples of suitable excipients include: diluents, binders, disintegrants, surfactants, hydrophilic polymers, film-coating polymers, lubricants, glidants (or anti-adherents), plasticizers, pH modifiers, preservatives, coloring, flavoring and/or aromatic substances.

There are applications in which the selected therapeutic agent is administered in small doses. In such applications, diluents are used to increase the volume occupied by a unit dose of the therapeutic agent so that the oral dosage form containing the therapeutic agent does not have inconveniently small dimensions. Diluents are selected in such applications so as to not inhibit the bioavailability of the therapeutic agent or impair the mechanical characteristics of the oral dosage form that is used. Examples of suitable diluents include microcrystalline cellulose; lactose, sucrose, fructose, glucose, dextrose, or other sugars; dibasic calcium phosphate; calcium sulfate; cellulose; ethylcellulose; cellulose derivatives; kaolin; mannitol, lactitol, maltitol, xylitol, sorbitol, or other sugar alcohols; dry starch; dextrin, maltodextrin or other polysaccharides; inositol; or mixtures thereof.

A binder may be used in the composition of the present invention to improve the physical properties of a oral dosage forms such as tablets made therefrom. In those applications in which the therapeutic agent has low bioavailability, a relatively large quantity of the therapeutic agent is required in each unit dose. When the therapeutic agent also exhibits poor pressability (the ability to be pressed into tablet form), such compositions yield oral dosage forms which have low strength and are unacceptably friable. The addition of a binder material to such compositions imparts cohesiveness to the composition and improves its physical properties. Examples of suitable binders include starches, hydroxypropylmethyl cellulose, hydroxypropyl cellulose, ethyl cellulose, polyvinyl pyrrolidone, acacia, guar gum, hydroxyethylcellulose, agar, calcium carrageenan, sodium alginate, gelatin, saccharides (including glucose, sucrose, dextrose and lactose), molasses, extract of Irish moss, panwar gum, ghatti gum, mucilage of isapol husk, carboxymethylcellulose, methylcellulose, veegum, larch arbolactan, polyethylene glycols, waxes and mixtures thereof.

A disintegrant may be used in the composition of the present invention to promote rapid decomposition of an oral dosage form thereof following administration. Rapid decomposition of the oral dosage form insures that the therapeutic agent is released quickly following administration. By providing a component in an oral dosage form which is readily soluble in the oral cavity, the oral dosage form is readily disintegrated when administered. Examples of suitable disintegrants include starches, sodium starch glycolate, crospovidone, croscarmellose, microcrystalline cellulose, low substituted hydroxypropyl cellulose, pectins, potassium methacrylate-divinylbenzene copolymer, polyvinyl alcohol, thylamide, sodium bicarbonate, sodium carbonate, starch derivatives, dextrin, beta cyclodextrin, dextrin derivatives, magnesium oxide, clays, bentonite and mixtures thereof.

A surfactant may be used in the composition of the present invention to disperse the therapeutic agent at the situs of release. Examples of suitable surfactants include nonionic surfactants, for example, sorbitan sesquioleate, polyoxyethylene sorbitan monooleate, polyoxyethylene monostearate, glycerol monostearate, propylene glycol monolaurate, polyoxyethylene lauryl ether, polyoxyethylene cetyl ether or polyoxyethylene hydrogenated castor oil, and ionic surfactants such as sodium dodecyl sulfate or benzalkonium chloride.

Examples of suitable hydrophilic polymers include hydroxypropylmethyl cellulose; carbomers; polyethylene oxides; hydroxypropyl cellulose; hydroxyethyl cellulose; carboxymethylcellulose; sodium carboxymethylcellulose; carboxyvinyl polymers; polyvinyl alcohol; glucans; scleroglucans; mannans; xanthans; carboxymethylcellulose and its derivatives; methylcellulose; cellulose; crosslinked polyvinylpyrrolidone; carboxymethyl starch; potassium methacrylate-divinylbenzene copolymer; hydroxypropylcyclodextrin; alpha, beta, gamma cyclodextrin or derivatives and other dextran derivatives; natural gums; seaweed extract; plant exudate; agar; agarose; algin; sodium alginate; potassium alginate; carrageenan; kappa-carrageenan; lambda-carrageenan; fucoidan, furcellaran; laminarin; hypnea; eucheuma; gum arabic; gum ghatti; gum karaya; gum tragacanth; guar gum; locust bean gum; quince psyllium; flax seed; okra gum; arabinogalactin; pectin; scleroglucan; dextran; amylose; amylopectin; dextrin; acacia; karaya; guar; a swellable mixture of agar and carboxymethyl cellulose; a swellable composition comprising methyl cellulose mixed with a sparingly cross-linked agar; a blend of sodium alginate; and locust bean gum.

Examples of suitable film-coating polymers include enteric polymer coating materials, such as, for example, cellulose acetate phthalate, cellulose acetate trimaletate, hydroxypropyl methylcellulose phthalate, polyvinyl acetate phthalate, Eudragit® poly acrylic acid and poly acrylate and methacrylate coatings, polyvinyl acetaldiethylamino acetate, hydroxypropyl methylcellulose acetate succinate, cellulose acetate trimellitate, shellac; hydrogels and gel-forming materials, such as, for example, carboxyvinyl polymers, sodium alginate, sodium carmellose, calcium carmellose, sodium carboxymethyl starch, polyvinyl alcohol, hydroxyethyl cellulose, methyl cellulose, gelatin, starch and cellulose-based cross-linked polymers, hydroxypropyl cellulose, hydroxypropyl methylcellulose, polyvinylpyrrolidone, crosslinked starch, microcrystalline cellulose, chitin, cellulose acetate, cellulose proprionate, cellulose acetate propionate, cellulose acetate butyrate, cellulose triacetate, aminoacryl-methacrylate copolymer (Eudragit® RS-PM, Rohm & Haas), pullulan, collagen, casein, agar, gum arabic, sodium carboxymethyl cellulose, carboxymethyl ethyl cellulose, swellable hydrophilic polymers, poly(hydroxyalkyl methacrylate) (m. wt. ~5k - 5,000k), polyvinylpyrrolidone (m. wt. ~10k - 360k), anionic and cationic hydrogels, polyvinyl alcohol having a low acetate residual, a swellable mixture of agar and carboxymethyl cellulose, copolymers of maleic anhydride and styrene, ethylene, propylene or isobutylene, pectin (m. wt. ~30k - 300k), polysaccharides such as agar, acacia, karaya, tragacanth, algins and guar, polyacrylamides, Polyox® polyethylene oxides (m. wt. ~100k - 5,000k), AquaKeep® acrylate polymers, diesters of polyglucan, crosslinked polyvinyl alcohol and poly N-vinyl-2-pyrrolidone, sodium starch glycollate (e.g. Explotab® ; Edward Mandell C. Ltd.); hydrophilic polymers such as polysaccharides, methyl cellulose, sodium or calcium carboxymethyl cellulose, hydroxypropyl methyl cellulose, hydroxypropyl cellulose, hydroxyethyl cellulose, nitro cellulose, carboxymethyl cellulose, cellulose ethers, poly(ethylene terphthalate), poly(vinyl isobutyl ether), polyurethane, polyethylene oxides (e.g. Polyox® , Union Carbide), methyl ethyl cellulose, ethylhydroxy ethylcellulose, cellulose acetate, ethylcellulose, cellulose butyrate, cellulose propionate, gelatin, collagen, starch, maltodextrin, pullulan, polyvinyl pyrrolidone, polyvinyl alcohol, polyvinyl acetate, glycerol fatty acid esters, polyacrylamide, polyacrylic acid, copolymers of methacrylic acid or methacrylic acid (e.g. Eudragit® , Rohm and Haas), other acrylic acid derivatives, ethyl acrylate-methyl methacrylate copolymer, sorbitan esters, polydimethyl siloxane, natural gums, lecithins, pectin, alginates, ammonia alginate, sodium, calcium, potassium alginates, propylene glycol alginate, agar, gums: arabic, karaya, locust bean, tragacanth, carrageenan, guar, xanthans, scleroglucan and mixtures and blends thereof.

A lubricant may be used in the composition of the present invention to facilitate manufacturing of oral dosage forms made therefrom. In applications in which the composition is tableted, a lubricant is included in the composition so that the pressed tablet will slide from the die in which it is pressed without mechanical damage to the tablet. Lubricants can also effect the manner in which individual components of a powder slide when pressed into tablets, reducing the formation of voids within a tablet, thereby reducing tablet to tablet weight variation due to void formation. Examples of suitable lubricants include stearic acid, magnesium stearate, talc, calcium stearate, hydrogenated vegetable oils, sodium benzoate, sodium chloride, leucine, magnesium lauryl sulfate, colloidal silicon dioxide, glyceryl mono stearate, waxes, hydrogenated oils, and polyethylene glycol.

Examples of suitable gliants (or anti-adherents) include colloidal silica, fumed silicon dioxide, silica hydrogels, talc, fumed silica, gypsum, kaolin, glycerol monostearate and magnesium stearate.

Suitable plasticizers include acetylated monoglycerides, butyl phthalyl butyl glycolate, dibutyl tartrate, diethyl phthalate, dimethyl phthalate, ethyl phthalyl ethyl glycolate, glycerin; propylene glycol, triacetin, citrate, tripropioin, diacetin, dibutyl phthalate, acetyl monoglyceride, polyethylene glycols, castor oil, triethyl citrate, polyhydric alcohols, glycerol, acetate esters, gylcerol triacetate, acetyl triethyl citrate, dibenzyl phthalate, dihexyl phthalate, butyl octyl phthalate, diisononyl phthalate, butyl octyl phthalate, dioctyl azelate, epoxidised tallate, triisoctyl trimellitate, diethylhexyl phthalate, di-n-octyl phthalate, di-i-octyl phthalate, di-i-decyl phthalate, di-n-undecyl phthalate, di-n-tridecyl phthalate, tri-2-ethylhexyl trimellitate, di-2-ethylhexyl adipate, di-2-ethylhexyl sebacate, di-2-ethylhexyl azelate, dibutyl sebacate, glyceryl monocaprylate, glyceryl monocaprate.

Examples of suitable pH modifiers include organic acids such as citric acid, fumaric acid, tartaric acid, succinic acid, ascorbic acid, malic acid, glutaric acid, adipic acid, lactic acid, fumaric acid, salts of these acids; salts of inorganic acids and magnesium hydroxide; sodium, potassium, calcium and magnesium salts of carbonate, bicarbonate, citrate and phosphate; and amino acids such as arginine, glycine and lysine.

Flavoring agents may be used in the compositions of the present invention to make oral dosage forms made therefrom more palatable than they would be in the absence of such flavoring agents. Unflavored oral dosage forms tend to be perceived as gritty or chalky in the absence of such flavoring agents. Flavoring agents play no essential part in the performance of the skim milk powder in masking the unpleasant taste of the therapeutic agent and, in fact, are ineffective by themselves in rendering palatable therapeutic agents which have an objectionable taste in the absence of skim milk powder. Suitable flavoring agents may provide sweetness, and/or a particular flavor, and include honey flavor, banana flavor, wild berry flavor, glycyrrhiza (licorice) flavor, chocolate flavor, vanilla flavor caramel flavor, and various forms of sugar.

The excipient is included in the composition in an amount sufficient to impart or enhance the physical and/or organoleptic properties of oral dosage formulations made therefrom. The amount of excipient will vary in accordance with a number of factors including, for example, the particular species of therapeutic agent used, the amount of skim milk powder used, the presence of other ingredients, the specific type of oral dosage formulation, and the particular application in which the composition is intended to be used. It is believed that for most applications, the amount of excipient included in the composition will be from about 0.1 to about 99 wt.%. In preferred embodiments, the amount of excipient included in the composition will be from about 0.2 wt.% to about 90 wt.%, and even more preferably from about 0.3 wt.% to about 70 wt.%.

Flowability refers to the ability of a bulk powder to fill a mold (as for pressing into a tablet). It is expressed as the tendency of a fixed volume of a powder to spread (rather than to form a conical shaped mound) when poured onto a flat surface under controlled conditions and is measured in units of degrees of angle of the conical mound with respect to the surface on which it sits. In preferred form, the composition of the present invention will have a flowability as measured by an angle of repose between about 30 to about 45.

Density is defined in the conventional sense of mass/unit volume. The bulk density of a powder composition includes the interparticulate void volume. As a result, the bulk density of a powder composition depends on both the density of the powder particles and the spatial arrangement of particles in the powder volume. In measuring the density of powders, bulk density is determined by measuring the volume of a known mass of powder that has been passed through a screen into a graduated cylinder. Tapped density is determined after a standard compacting vibration has been applied to the volumetric vessel. Lower density powders tend to be more easily pressed into cohesive tablets than high density powders, and low density powders tend to achieve cohesiveness at lower pressing pressure, and therefore generally also yield lower density tablets. In preferred form, the composition of the present invention has a bulk density of from about 0.4 to about 0.9 g/ml and a tapped density of from about 0.5 to about 1.1 g/ml.

The oral dosage formulation may be in the form of a chewable tablet, swallowable tablet, effervescent tablet, fast melt tablet.

In preferred embodiments, the oral dosage formulation is in the form of a chewable tablet or a fast melt tablet. In such embodiments, the tablet may be uncoated or it may be coated by known techniques for a variety of purposes including, for example, protection of the composition, or improving the aesthetics of the tablet.

Tablets can be characterized by homogeneity, hardness, friability, speed of disintegration, and speed of dispersibility of the therapeutic agent contained therein. Homogeneity refers to the variability of the amount of therapeutic agent contained in random samples of the bulk powder or dosage forms. Defined in terms of stated dose, it is preferred that any one sample of an oral dosage form of the present invention contain between about 85% and about 115% of the stated dose of the therapeutic agent. A 10 unit sampling of an oral dosage form of the present invention, when averaged, will provide preferably between about 95% and about 105% of the stated dose of the therapeutic agent.

Tablet hardness is expressed as the amount of force that must be applied for a tablet to break. One method of measuring tablet hardness employs an apparatus known as a Schleuniger tablet hardness tester. According to this method, a force is applied perpendicular to the edge of the tablet and directed across the diameter of the tablet under test until the tablet fractures. In preferred embodiments of the present invention in which the oral dosage form is a chewable tablet, the tablet has a hardness, as measured by a Schleuniger tablet hardness tester, of from about 0.5 Kp to about 30 Kp and more preferably from about 1 Kp to about 20 Kp. In preferred embodiments of the present invention in which the oral dosage form is a fast melt tablet, the tablet has a hardness, as measured by a Schleuniger tablet hardness tester, of from about 0.5 Kp to about 10 Kp and more preferably from about 1 Kp to about 10 Kp. In preferred embodiments of the present invention in which the oral dosage form is an effervescent tablet, the tablet has a hardness, as measured by a Schleuniger tablet hardness tester, of from about 1 Kp to about 25 Kp and more preferably from about 3 Kp to about 20 Kp.

Friability is measured by tumbling a standard sampling of tablets in a standard apparatus at a standard speed for a standard time and measuring the weight loss of the tablets due to material being ablated from the tablets. In preferred embodiments of the present invention in which the oral dosage form is a tablet, the tablet has a friability of from about 0.1% to about 3%, and more preferably from about 0.2% to about 1%.

Disintegration time is measured by placing the tablet in a standard gastric solution held at a given temperature with a standard amount of agitation. The time recorded is the time elapsed from contact with the solution until the tablet falls into pieces small enough to pass through a standard screen. In preferred embodiments of the present invention in which the oral dosage form is a non-effervescent tablet, the tablet has a disintegration time of from about 10 seconds to about 30 minutes and more preferably from about 30 seconds to about 15 minutes. In preferred embodiments of the oral dosage form of the present invention in which the oral dosage form is in the form of an effervescent tablet, the tablet will have a disintegration time from about 10 seconds to about 5 minutes, and more preferably from about 30 to about 3 minutes.

The composition of the present invention are made by dry blending the therapeutic agent and skim milk powder and optional ingredients to homogeneity. The dry blended composition is suitable for use in forming the oral dosage formulations of the present invention and, upon ingestion, will mask effectively the unpleasant taste of the therapeutic agent.

### EXAMPLES

The following compositions are illustrative of the taste-masking effect of the present invention as applied to a variety of therapeutic agents which are recognized as having an unpleasant taste. The various sugars, sweeteners, and flavoring agents employed in the compositions described below are insufficient on their own to eliminate the unpleasant taste of the therapeutic agents with which they are combined, but serve only to enhance further the taste of the resulting composition and/or the physical properties of the oral dosage forms made therefrom.

The oral dosage forms described in Example Nos. 1 to 12 set forth below were evaluated for their taste. The taste test was designed to elicit subjective evaluations of taste with respect to various flavor characteristics such as sweet, salty, sour, bitter, warm, cool, burning, irritating, anesthetic, and aftertaste. A group of volunteer tasters were provided a series of samples and asked to rate each sample on a scale from 1 (lowest) to 5 (highest) for each taste characteristic. The samples that were provided comprised both unmasked therapeutic agents as well as oral dosage formulations in accordance with the present invention that included such therapeutic agents in combination with skim milk powder. The data collected from the volunteers (set out in tabular form in and found to provide a statistically significant change in taste ratings when comparing the unmasked therapeutic agent to the therapeutic agent as part of oral dosage formulations made in accordance with the present invention.

For Example Nos. 1 through 11, the ingredients were blended mechanically for 15 minutes at room temperature in a cube mixer. The resulting mixtures were tableted by a single punch tableting machine using a plane punch of 18 mm in diameter.

### Example No. 1

In this example, the unpleasant chalky taste associated with calcium phosphate dibasic has been is eliminated effectively by the inclusion of skim milk powder in the tablet described below.

| | |
|---|---|
| calcium phosphate dibasic | 3.90 g |
| skim milk powder | 135.55 g |
| sorbitol | 108.70 g |
| honey flavor | 0.76 g |
| banana flavor | 1.09 g |
| magnesium stearate | 0.76 g |

The tablets produced in this example had a thickness of approximately 7.7 mm and a hardness of 23 Kp as measured by a Schleuniger tablet hardness tester, weighed 2.3 g each, contained 10.6 mg of calcium and 8.1 mg of phosphorous per tablet, and had a pleasant and sweet taste when dissolved in the oral cavity.

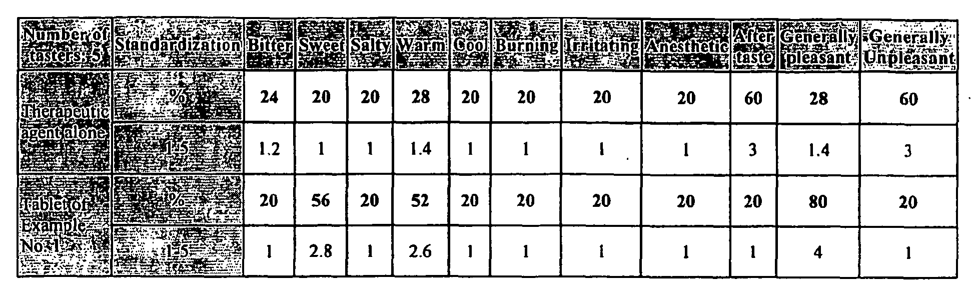

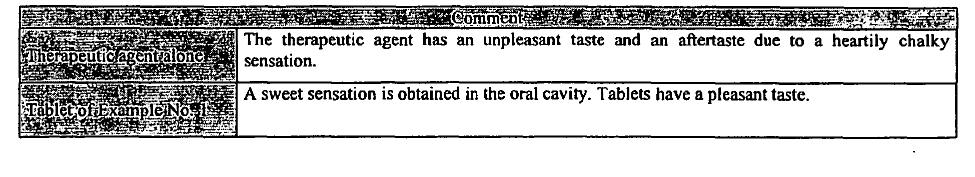

### Example No. 2

In this example, the taste-masking effect is demonstrated in a tablet that includes two therapeutic agents, namely, calcium phosphate dibasic and vitamin D₃ which is known to have a strong and unpleasant vitamin taste.

| | |
|---|---|
| calcium phosphate dibasic | 3.12 g |
| vitamin D₃ | 0.17 g |
| skim milk powder | 109.14 g |
| dextrose | 86.96 g |
| magnesium stearate | 0.61 g |

The tablets produced in this example had a thickness of approximately 7.8 mm and a hardness of 11 Kp as measured by a Schleuniger tablet hardness tester, weighed 2.3 g each, contained 10.6 mg of calcium, 8.1 mg of phosphorus, and 5µg of vitamin D₃ per tablet, and had a pleasant taste when dissolved in the oral cavity.

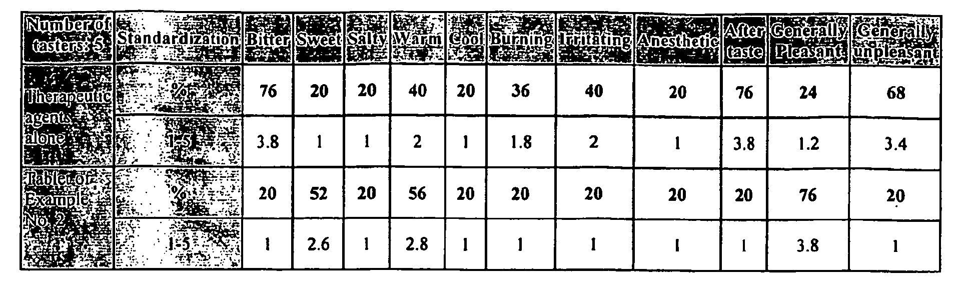

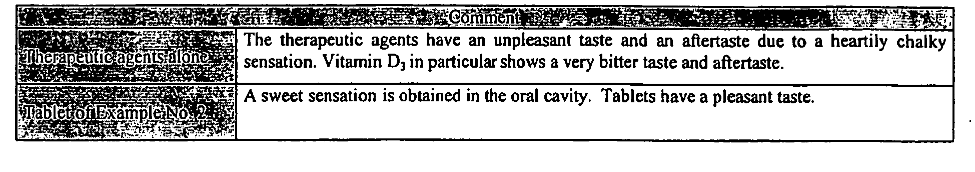

### Example No. 3

In this example, the taste-masking effect is demonstrated by a tablet that includes calcium phosphate dibasic and acerola which is an excellent source of vitamin C and is recognized as having an unpleasant acidic taste.

| | |
|---|---|
| calcium phosphate dibasic | 3.90 g |
| acerola | 38.37 g |
| skim milk powder | 97.18 g |
| fructose | 108.70 g |
| wild berry flavor | 1.09 g |
| magnesium stearate | 0.76 g |

The tablets produced in this example had a thickness of approximately 7.5 mm and a hardness of 27 Kp as measured by a Schleuniger tablet hardness tester, weighed 2.3 g each, contained 10.6 mg of calcium, 8.1 mg of phosphorus, and 60 mg of vitamin C per tablet, and had a pleasant taste when dissolved in the oral cavity.

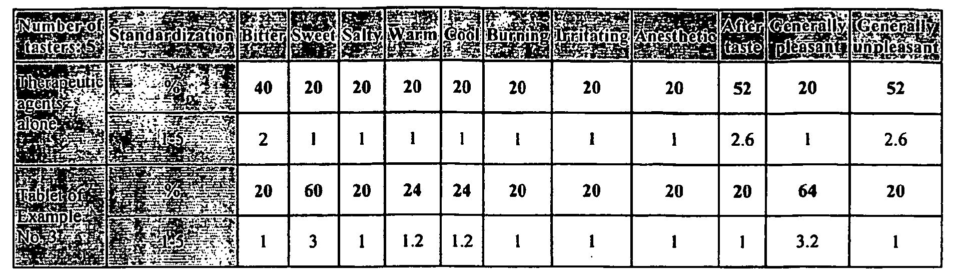

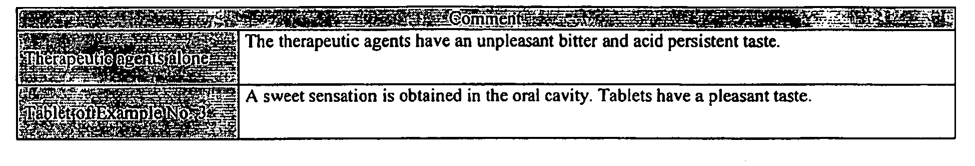

### Example No. 4

This example demonstrates the taste-masking effect in a tablet that includes a combination of vitamins each of which is known to have a strong and unpleasant vitamin taste.

| | |
|---|---|
| vitamin B₁ mononitrate | 0.07 g |
| vitamin B₆ | 0.09 g |
| vitamin PP | 0.82 g |
| skim milk powder | 57.80 g |
| fructose | 40.90 g |
| magnesium stearate | 0.32 g |

The tablets produced in this example had a thickness of approximately 7.7 mm and a hardness of 20 Kp as measured by a Schleuniger tablet hardness tester, weighed 2.3 g each, contained 1.50 mg of vitamin B₁, 2.00 mg of vitamin B₆ and 18.00 mg of vitamin PP per tablet, and had a pleasant taste when dissolved in the oral cavity.

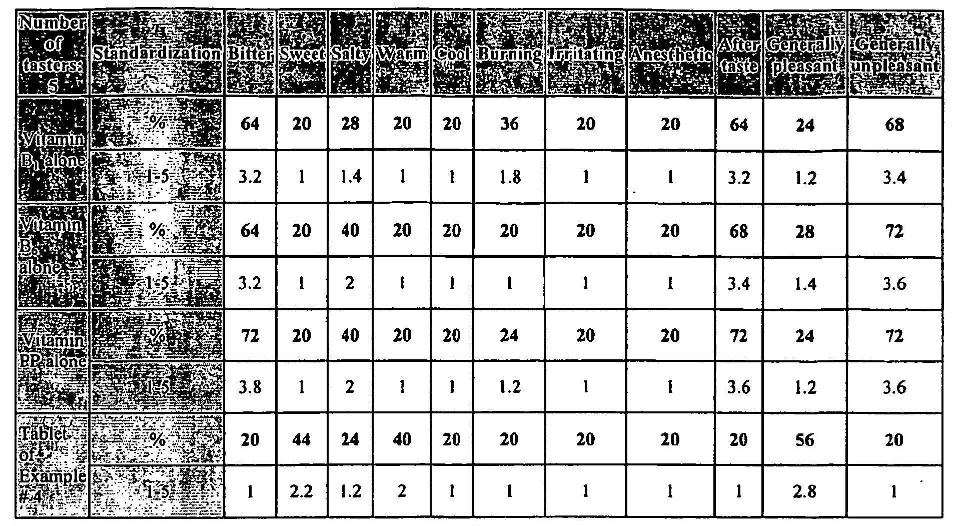

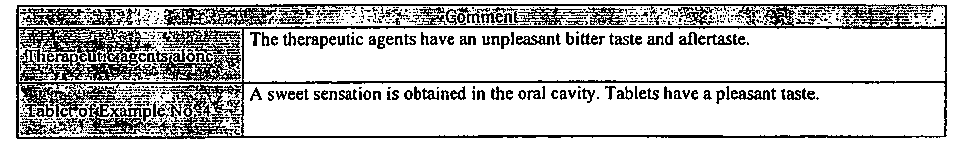

### Example No. 5

This example demonstrates the taste-masking effect in a tablet that includes ginseng extract which is known to have an unpleasant bitter taste.

| | |
|---|---|
| ginseng extract | 4.55 g |
| skim milk powder | 54.00 g |
| sorbitol | 40.90 g |
| glycirriza flavor | 0.23 g |
| magnesium stearate | 0.32 g |

The tablets produced in this example had a thickness of approximately 7.4 mm and a hardness of 22 Kp as measured by a Schleuniger tablet hardness tester, weighed 2.2 g each, contained 100.00 mg of ginseng extract per tablet, and had a pleasant taste when dissolved in the oral cavity.

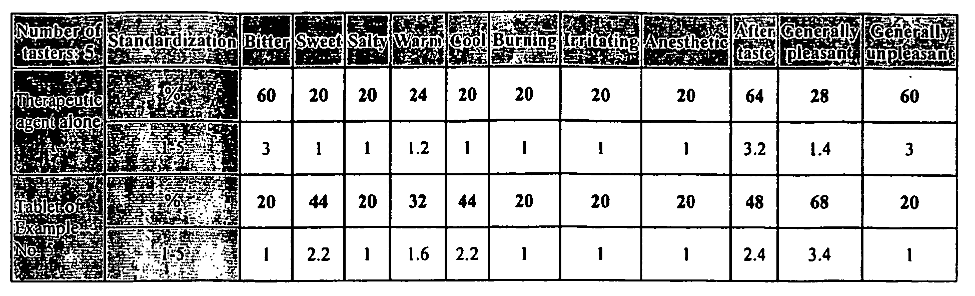

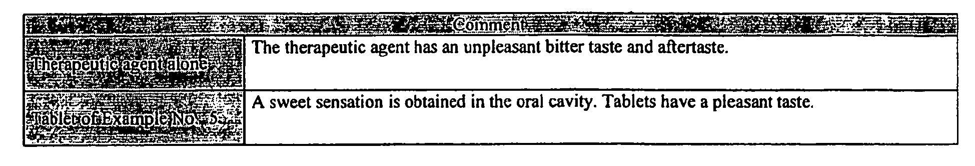

### Example No. 6

This example demonstrates the taste-masking effect in a tablet that includes guarana extract, another therapeutic agent which is known to have an unpleasant bitter taste.

| | |
|---|---|
| guarana extract | 2.27 g |
| skim milk powder | 56.05 g |
| dextrose | 40.90 g |
| white chocolate flavor | 0.46 g |
| magnesium stearate | 0.32 g |

The tablets produced in this example had a thickness of approximately 7.4 mm and a hardness of 23 Kp as measured by a Schleuniger tablet hardness tester, weighed 2.2 g each, contained 50.00 mg of guarana extract per tablet, and had a pleasant taste when dissolved in the oral cavity.

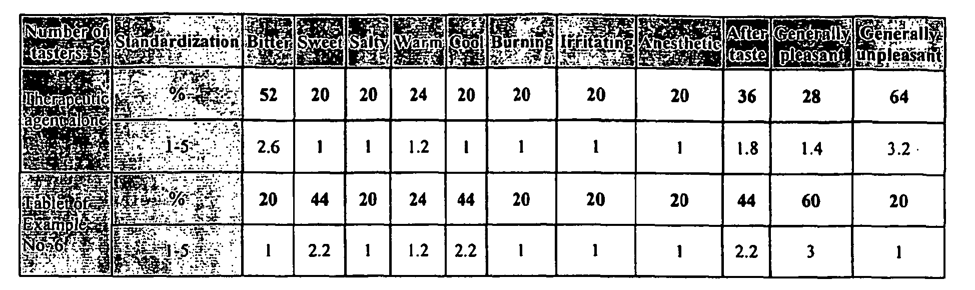

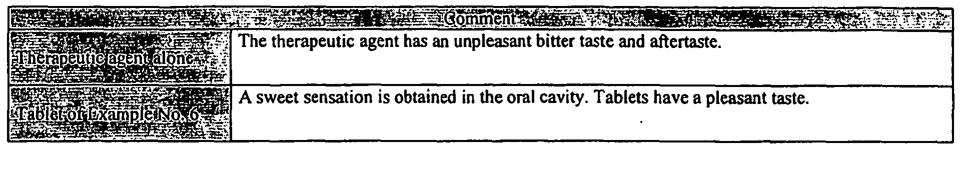

### Example No. 7

This example demonstrates the taste-masking effect in a tablet that includes clemastine fumarate, a pharmaceutical agent which is known to have an unpleasant bitter taste.

| | |
|---|---|
| clemastine fumarate | 0.09 g |
| skim milk powder | 58.23 g |
| sorbitol | 40.90 g |
| white chocolate flavor | 0.46 g |
| magnesium stearate | 0.32 g |

The tablets produced in this example had a thickness of approximately 7.4 mm and a hardness of 25 Kp as measured by a Schleuniger tablet hardness tester, weighed 2.2 g each, contained 2.00 mg of clemastine fumarate per tablet, and had a pleasant taste when dissolved in the oral cavity.

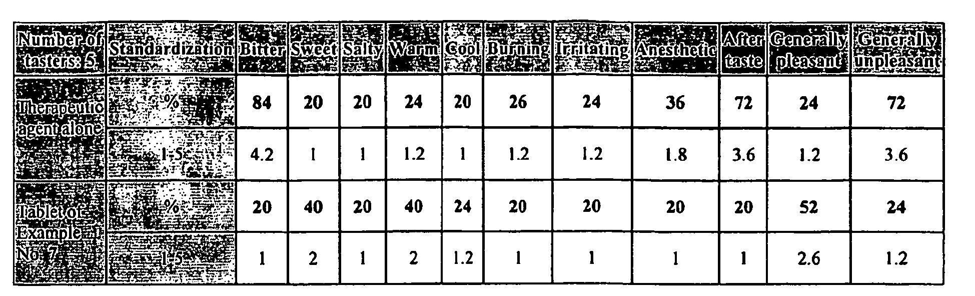

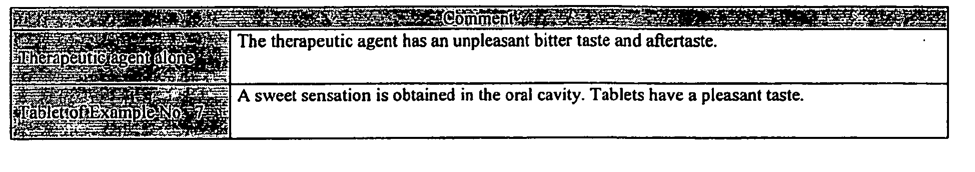

### Example No. 8

This example demonstrates the taste-masking effect in a tablet that includes cetirizine di-HCl, a pharmaceutical agent which is known to have an unpleasant bitter taste.

| | |
|---|---|
| cetirizine di-HCl | 0.45 g |
| skim milk powder | 57.87 g |
| dextrose | 40.90 g |
| white chocolate flavor | 0.46 g |
| magnesium stearate | 0.32 g |

The tablets produced in this example had a thickness of approximately 7.4 mm and a hardness of 18 Kp as measured by a Schleuniger tablet hardness tester. The resulting tablets weighed 2.2 g. each, contained 10.00 mg. of cetirizine di-HCl per tablet, and had a pleasant taste when dissolved in the oral cavity.

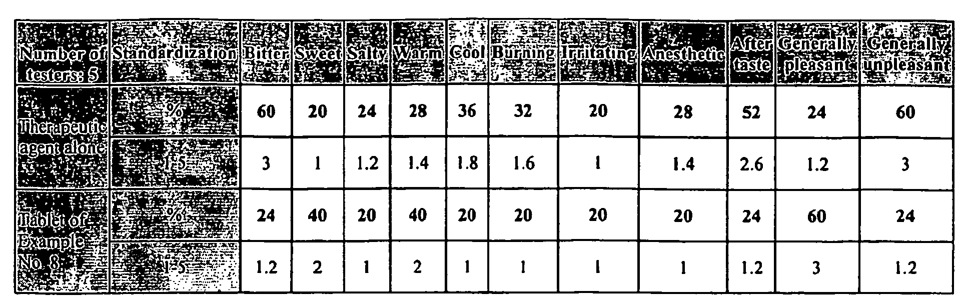

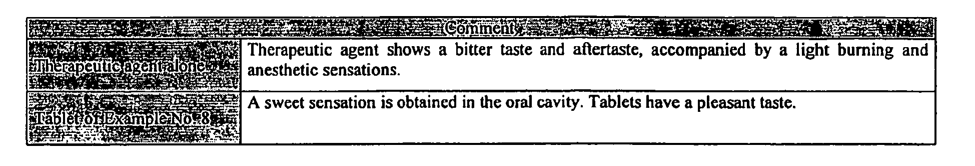

### Example No. 9

This example demonstrates the taste-masking effect in a tablet that includes famotidine, a pharmaceutical agent which is known to have an unpleasant bitter taste.

| | |
|---|---|
| famotidine | 0.91 g |
| skim milk powder | 57.41 g |
| saccharose | 40.90 g |
| white chocolate flavor | 0.46 g |
| magnesium stearate | 0.32 g |

The tablets produced in this example had a thickness of approximately 7.4 mm and a hardness of 18 Kp as measured by a Schleuniger tablet hardness tester, weighed 2.2 g each, contained 20.00 mg of famotidine, and had a pleasant taste when dissolved in the oral cavity.

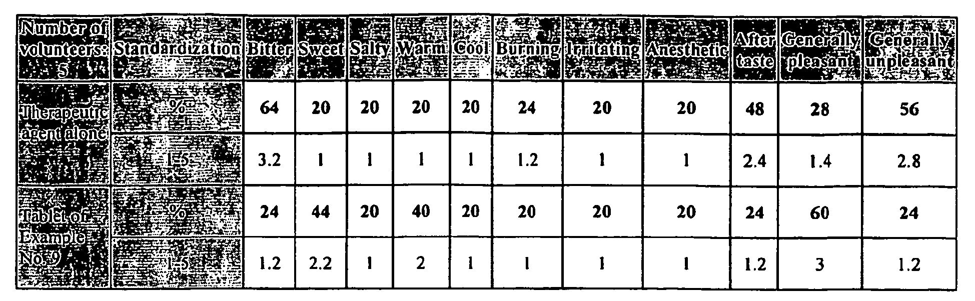

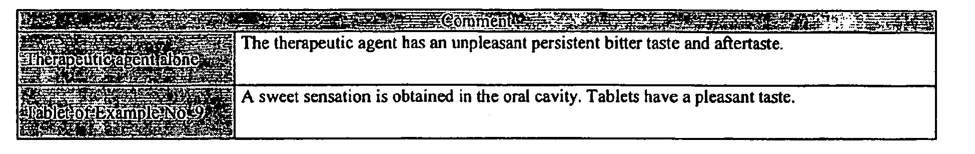

### Example No. 10

This example demonstrates the taste-masking effect in a tablet that includes prednisolone sodium phosphate, a pharmaceutical agent which is known to have an unpleasant bitter taste.

| | |
|---|---|
| prednisolone sodium phosphate | 2.73 g |
| skim milk powder | 55.82 g |
| fructose | 40.90 g |
| glycyrrhiza flavor | 0.23 g |
| magnesium stearate | 0.32 g |

The tablets produced in this example had a thickness of approximately 7.4 mm and a hardness of 20 Kp as measured by a Schleuniger tablet hardness tester, weighed 2.2 g each, contained 60.00 mg of prednisolone sodium phosphate, and had a pleasant taste when dissolved in the oral cavity.

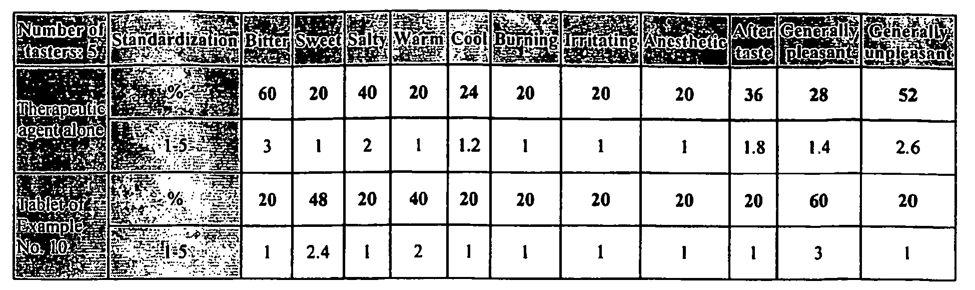

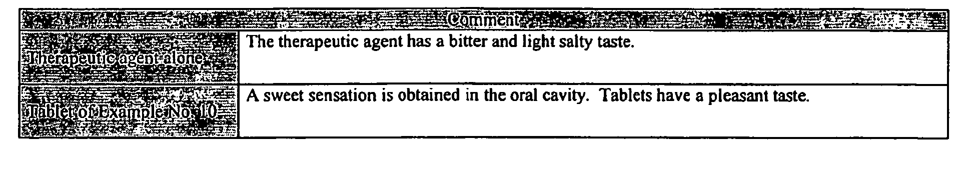

### Example No. 11

This example demonstrates the taste-masking effect in a tablet that includes imipramine HCl, a pharmaceutical agent which is known to have an unpleasant bitter taste.

| | |
|---|---|
| imipramine HCl | 1.14 g |
| skim milk powder | 57.41 g |
| fructose | 40.90 g |
| glycyrrhiza flavor | 0.23 g |
| magnesium stearate | 0.32 g |

The tablets produced in this example had a thickness of approximately 7.4 mm and a hardness of 23 Kp as measured by a Schleuniger tablet hardness tester, weighed 2.2 g each, contained 25.00 mg of imipramine HCl, and had a pleasant taste when dissolved in the oral cavity.

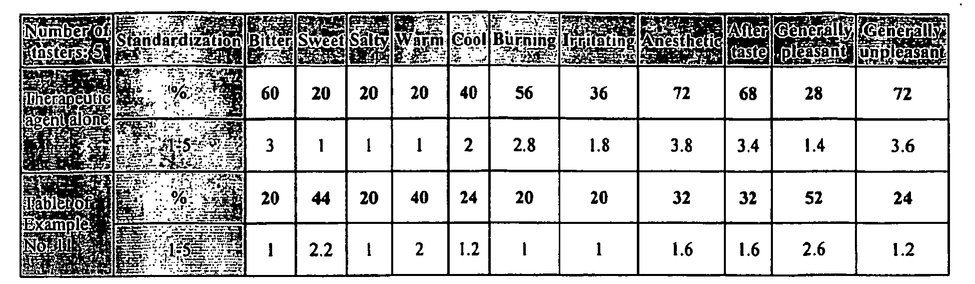

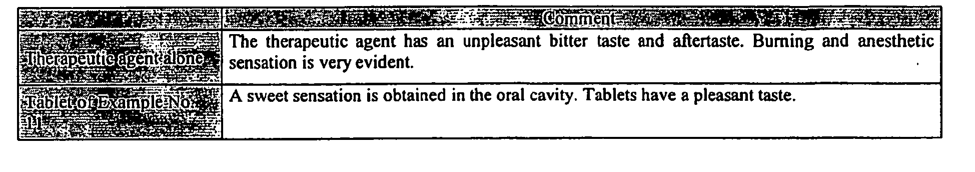

### Example No. 12

This example demonstrates the taste-masking effect in a tablet that includes vitamin PP, a therapeutic agent which is known to have an unpleasant taste.

| | |
|---|---|
| vitamin PP | 15.00 g |
| skim milk powder | 213.33 g |
| aspartame | 6.67 g |
| vanilla flavor | 8.34 g |
| magnesium stearate | 6.67 g |

The above ingredients were blended mechanically for 15 minutes at room temperature in a cube mixer. The resulting mixture was tableted by a single punch tableting machine using a plane punch of 12 mm in diameter to yield tablets with a thickness of approximately 3 mm and a hardness of 7.5 Kp as measured by a Schleuniger tablet hardness tester. The resulting tablets weighed 300 mg each, contained 18.00 mg of vitamin PP, and had a pleasant taste when dissolved in the oral cavity.

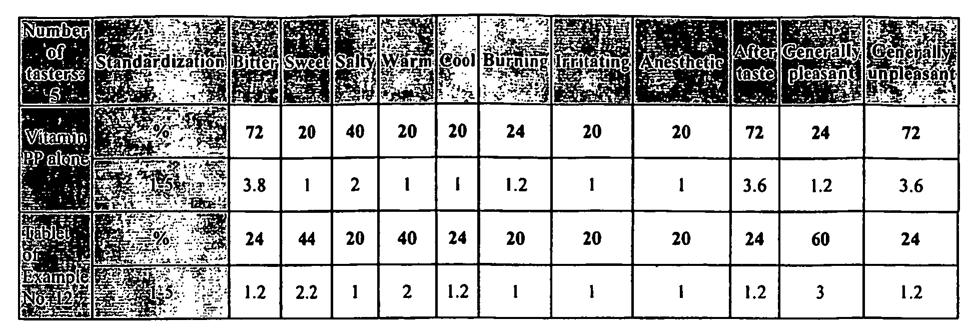

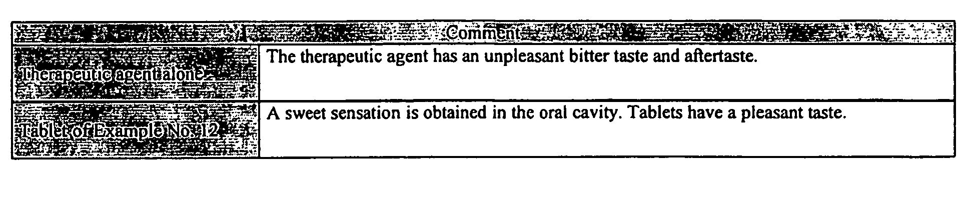

For Example Nos. 13 through 16, the ingredients were blended mechanically for 20 minutes at room temperature in a cube mixer. The resulting mixtures were tableted by a single punch tableting machine using a plane punch of 12 mm in diameter to yield tablets with a thickness of approximately 3 mm and a hardness of about 6 Kp as measured by a Schleuniger tablet hardness tester. The resulting tablets weighed 300 mg each, contained 18.00 mg of vitamin PP, and had a pleasant taste when dissolved in the oral cavity.

### Example No. 13

This example demonstrates the taste-masking effect in a chewable tablet that includes vitamin PP, a therapeutic agent which is known to have an unpleasant taste, and pregelatinized starch.

| | |
|---|---|
| vitamin PP | 7.50 g |
| skim milk powder | 100.42 g |
| aspartame | 3.34 g |
| vanilla flavor | 4.17 g |
| pregelatinized starch | 6.25 g |
| magnesium stearate | 3.34 g |

### Example No. 14

This example also demonstrates the taste-masking effect in a chewable tablet that includes vitamin PP, and a polyethylene oxide resin.

| | |
|---|---|
| vitamin PP | 7.50 g |
| skim milk powder | 104.17 g |
| aspartame | 3.34 g |
| vanilla flavor | 4.17 g |
| polyethylene oxide resin | 2.50 g |
| magnesium stearate | 3.34 g |

### Example No. 15

This example also demonstrates the taste-masking effect in a chewable tablet that includes vitamin PP and hydroxypropyl methylcellulose.

| | |
|---|---|
| vitamin PP | 15.00 g |
| skim milk powder | 171.67 g |
| aspartame | 6.67 g |
| vanilla flavor | 8.34 g |
| hydroxypropyl methylcellulose | 41.67 g |
| magnesium stearate | 6.67 g |

### Example No. 16

This example also demonstrates the taste-masking effect in a chewable tablet that includes vitamin PP and gum arabic.

| | |
|---|---|
| vitamin PP | 0.18 g |
| skim milk powder | 1.71 g |
| gum arabic | 1.41 g |

### Example No. 17

This example also demonstrates the taste-masking effect in a chewable tablet that includes calcium carbonate.

| | |
|---|---|
| Cal-carb 4450PG (91.6%) | 2729.26 g |
| skim milk powder | 1000 g |
| aspartame | 20 g |
| magnesium stearate | 28 g |
| vanilla flavor | 22.74 g |

Cal-carb 4450PG (91.6%) is a calcium carbonate supplied by CHR Hansen, Inc. of Vineland, NJ, that contains 91.6 % in calcium carbonate with the balance comprising maltodextrin and pregelatinized starch. The above ingredients were blended mechanically for 5 minutes at room temperature in a cube mixer to homogeneous granules. The resulting mixture was tableted by a single punch tableting machine to yield tablets with a thickness of approximately 9 mm and a hardness of about 9 Kp as measured by a Schleuniger tablet hardness tester. The resulting tablets weighed 3.8 g each, contained 1000 mg of calcium carbonate, and had a pleasant taste when chewed.

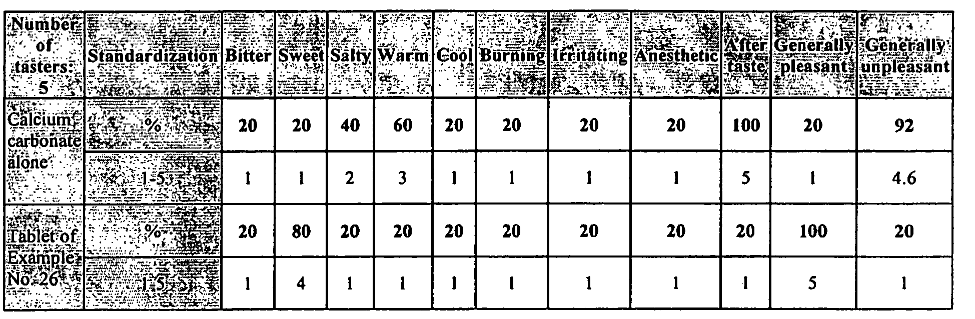

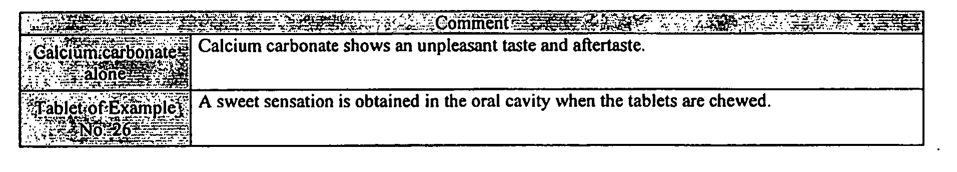

### Example No. C-1

This comparative example demonstrates the unacceptable properties of a vitamin PP-containing tablet that includes albumin, a protein found in skim milk powder.

| | |
|---|---|
| albumin | 71.11 g |
| vitamin PP | 5 g |
| aspartame | 2.22 g |
| vanilla flavor | 2.78 g |
| magnesium stearate | 2.22 g |

The above ingredients were blended mechanically to homogeneity for about 15 minutes at about room temperature in a cube mixer. The resulting mixture was tableted by a single punch tableting machine using a plane punch of 12 mm in diameter to yield tablets with a thickness of approximately 12mm and a hardness of about 0.1 Kp as measured by a Schleuniger tablet hardness tester. The resulting tablets weighed 300 mg each and contained 18 mg of vitamin PP. It was noted that the tablets were unacceptably friable due to the poor binding properties of albumin.

## Claims

1. A method of producing a pharmaceutical composition in the form of chewable, swallowable, effervesecents or fast-melt tablets which composition comprises a therapeutically effective amount of a therapeutic agent having an unpleasant taste and skim milk powder having a fat content of no more than 1.25% in an amount sufficient to mask the unpleasant taste of the therapeutic agent, which method comprises the step of dry blending the therapeutic agent having an unpleasant taste and skim milk powder in an amount sufficient to mask the unpleasant taste of the therapeutic agent, and tableting the resulting mixture.

2. A pharmaceutical composition in form of chewable, swallowable, effervescent or fast-melt tablets comprising a therapeutically effective amount of a therapeutic agent having an unpleasant taste and skim milk powder having a fat content of no more than 1.25% in an amount sufficient to mask the unpleasant taste of the therapeutic agent, which pharmaceutical compositions is obtainable by a method which comprises the step of dry blending the therapeutic agent and skim milk powder in an amount sufficient to mask the unpleasant taste of the therapeutic agent, and tableting the resulting mixture.

3. A pharmaceutical composition according to claim 2 which in addition comprises a disintegrant.

4. A composition of claim 2 or 3 further comprising a pharmaceutical excipient.

5. Use of a skim milk powder as a taste-masking agent in a pharmaceutical composition which pharmaceutical composition is obtainable by dry blending a therapeutic agent having an unpleasant taste and the skim milk powder, and tableting the resulting mixture.

6. Use according to claim 5, wherein the pharmaceutical composition has been obtained by dry blending a therapeutic agent having an unpleasant taste and the skim milk powder.

## Patentansprüche

1. Verfahren zur Herstellung einer pharmazeutischen Zusammensetzung in Form von Kau-, Schluck-, Brause- oder schnellschmelzenden Tabletten, wobei die Zusammensetzung eine therapeutisch wirksame Menge eines Therapeutikums mit einem unangenehmen Geschmack und Magermilchpulver mit einem Fettgehalt von nicht mehr als 1,25% in einer ausreichenden Menge, um den unangenehmen Geschmack des Therapeutikums zu überdecken, umfasst, wobei das Verfahren den Schritt des trockenen Vermischens des Therapeutikums mit dem unangenehmen Geschmack und des Magermilchpulvers in einer ausreichenden Menge, um den unangenehmen Geschmack des Therapeutikums zu überdecken, und das Tablettieren des resultierenden Gemischs umfasst.

2. Pharmazeutische Zusammensetzung in Form von Kau-, Schluck-, Brause- oder schnellschmelzenden Tabletten, wobei die Zusammensetzung eine therapeutisch wirksame Menge eines Therapeutikums mit einem unangenehmen Geschmack und Magermilchpulver mit einem Fettgehalt von nicht mehr als 1,25% in einer ausreichenden Menge, um den unangenehmen Geschmack des Therapeutikums zu überdecken, umfasst, wobei die pharmazeutische Zusammensetzung nach einem Verfahren erhältlich ist, das den Schritt des trockenen Vermischens des Therapeutikums und des Magermilchpulvers in einer ausreichenden Menge, um den unangenehmen Geschmack des Therapeutikums zu überdecken, und das Tablettieren des resultierenden Gemischs umfasst.

3. Pharmazeutische Zusammensetzung gemäß Anspruch 2, die weiterhin ein Sprengmittel umfasst.

4. Zusammensetzung gemäß Anspruch 2 oder 3, die weiterhin einen pharmazeutischen Trägerstoff umfasst.

5. Verwendung eines Magermilchpulvers als geschmacksüberdeckendes Mittel in einer pharmazeutischen Zusammensetzung, wobei die pharmazeutische Zusammensetzung durch trockenes Vermischen eines Therapeutikums mit einem unangenehmen Geschmack und des Magermilchpulvers und Tablettieren des resultierenden Gemischs erhältlich ist.

6. Verwendung gemäß Anspruch 5, wobei die pharmazeutische Zusammensetzung durch trockenes Vermischen eines Therapeutikums mit einem unangenehmen Geschmack und des Magermilchpulvers erhalten wurde.

## Revendications

1. Méthode de production d'une composition pharmaceutique sous la forme de comprimés effervescents ou fondant rapidement, croquables, pouvant être avalés, la composition comprenant une quantité thérapeutiquement efficace d'un agent thérapeutique ayant un goût déplaisant et de poudre de lait écrémé ayant un taux de matière grasse ne dépassant pas 1,25 % en une quantité suffisante pour masquer le goût déplaisant de l'agent thérapeutique, ladite méthode comprenant l'étape de mélange à sec de l'agent thérapeutique ayant un goût déplaisant et de poudre de lait écrémé en une quantité suffisante pour masquer le goût déplaisant de l'agent thérapeutique, et de compression du mélange obtenu.

2. Composition pharmaceutique sous la forme de comprimés effervescents ou fondant rapidement, croquables, pouvant être avalés, comprenant une quantité thérapeutiquement efficace d'un agent thérapeutique ayant un goût déplaisant et de poudre de lait écrémé ayant un taux de matière grasse ne dépassant pas 1,25 % en une quantité suffisante pour masquer le goût déplaisant de l'agent thérapeutique, ladite composition pharmaceutique pouvant être obtenue par une méthode comprenant l'étape de mélange à sec de l'agent thérapeutique et de poudre de lait écrémé en une quantité suffisante pour masquer le goût déplaisant de l'agent thérapeutique, et de compression du mélange obtenu.

3. Composition pharmaceutique selon la revendication 2, qui comprend en outre un agent de délitement.

4. Composition selon la revendication 2 ou 3 comprenant en outre un excipient pharmaceutique.

5. Utilisation d'une poudre de lait écrémé pour masquer un goût déplaisant dans une composition pharmaceutique, cette composition pharmaceutique pouvant être obtenue par mélange à sec d'un agent thérapeutique ayant un goût déplaisant et de la poudre de lait écrémé, et de compression du mélange obtenu.

6. Utilisation selon la revendication 5, dans laquelle la composition pharmaceutique a été obtenue par mélange à sec d'un agent thérapeutique ayant un goût déplaisant et de poudre de lait écrémé.
